# EUROPEAN PATENT APPLICATION

(11) **EP 4 474 018 A1**
(43) Date of publication of application: **11.12.2024**
(21) Application number: 23749543.7
(22) Date of filing: 20.01.2023
(51) Int. Cl.: A61P 43/00, A61P 17/16, A61Q 19/08, A61K 8/49, A61K 8/60, A61K 31/4015, A61K 31/4166, A61K 31/519

(54) **SKIN CARE COMPOSITION**

(30) Priority: 03.02.2022 JP 2022015836
(71) Applicant: Shiseido Company, Ltd., Chuo-ku Tokyo 104-0061 (JP)
(72) Inventor: SUZUKI Ikuhiro, Tokyo 104-0061 (JP)
(74) Representative: Ter Meer Steinmeister & Partner
(86) International application number: PCT/JP2023/001625
(87) International publication number: WO 2023/149227

(57) **Abstract**

[Object] Provided is an external-use skin preparation composition that effectively inhibits elastase activity.

[Solution] An external-use skin preparation composition comprising (A) a cyclic carboxamide derivative having a specific structure or a salt thereof, and (B) adenosine, an adenosine derivative or a salt thereof.

## Description

### TECHNICAL FIELD

The present invention relates to an external-use skin preparation composition comprising a cyclic carboxamide derivative having a specific structure or a salt thereof, and adenosine, an adenosine derivative or a salt thereof.

### BACKGROUND ART

The cyclic carboxamide derivative has an effect of inhibiting heparanase activity, and for example, it has been proposed that the cyclic carboxamide derivative be blended into a cosmetic as a wrinkle ameliorating agent or as a whitening agent effective for preventing or suppressing pigmentation such as pigmented macules (Patent Literature 1).

Adenosine is known to have an effect of promoting blood circulation when applied to skin, and is applied to an external preparation for skin such as a hair restorer (Patent Literature 2).

### CITATION LIST

### PATENT LITERATURE

Patent Literature 1: WO 2011/040496
Patent Literature 2: JP 2008-247754 A

It is considered that elastase activity of degrading elastic fibers (elastin) produced by dermal fibroblasts is involved in the decrease in skin elasticity such as wrinkles.

According to the study of the present inventors, it has been surprisingly found that a composition comprising a cyclic carboxamide derivative or a salt thereof in combination with adenosine, an adenosine derivative or a salt thereof effectively inhibits elastase activity. The present invention is based on these findings.

According to the present invention, the following invention is provided.
[1] An external-use skin preparation composition comprising:
   (A) a cyclic carboxamide derivative represented by Formula (1) or a salt thereof 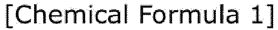 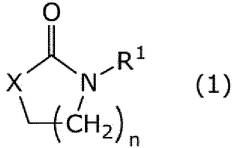 (in the formula,
      R¹ is a hydrocarbon group having 1 to 6 carbon atoms which can be substituted with a hydroxy group, or a hydrogen atom,
      X is -CH₂- or -N(R²)-, where R² is a hydrocarbon group having 1 to 6 carbon atoms which can be substituted with a hydroxy group, or a hydrogen atom, and
      n is an integer of 1 to 3); and
   (B) adenosine, an adenosine derivative or a salt thereof.
[2] The composition according to [1],
   in which, in Formula (1) of the component (A),
   R¹ is a hydroxyalkyl group having 1 to 3 carbon atoms,
   X is -CH₂- or -NH-, and
   n is 1.
[3] The composition according to [1] or [2], in which the component (A) is 1-(2-hydroxyethyl)-2-imidazolidinone.
[4] The composition according to any one of [1] to [3], in which a blending amount of the component (A) is 7.5 to 200 mg/mL.
[5] The composition according to any one of [1] to [4], in which the component (B) is adenosine, adenosine 5'-phosphate, or a salt of adenosine 5'-phosphate.
[6] The composition according to any one of [1] to [5], in which the component (B) is adenosine.
[7] The composition according to any one of [1] to [5], in which a blending amount of the component (B) is 0.5 to 10 mg/mL.
[8] The composition according to any one of [1] to [7], which is an anti-aging cosmetic.
[9] The composition according to any one of [1] to [8], which is an anti-wrinkle cosmetic.
[10] The composition according to any one of [1] to [9], in which the composition has elastase inhibitory activity.

According to the present invention, it is possible to provide an external-use skin preparation composition that effectively inhibits elastase activity.

### BEST MODE FOR CARRYING OUT THE INVENTION

The present invention relates to an external-use skin preparation composition (hereinafter, can be referred to as a composition) comprising (A) a cyclic carboxamide derivative having a specific structure or a salt thereof, and (B) adenosine, an adenosine derivative or a salt thereof.

In general, wrinkles and sagging are caused by aging and photoaging. One of causes of wrinkles and sagging is a decrease in skin elasticity, and it is considered that elastase activity of degrading elastic fibers (elastin) produced by dermal fibroblasts relates thereto. The composition according to the present invention has elastase inhibitory activity and can effectively inhibit elastase activity. As a result, the degradation of elastin is suppressed, and wrinkles, sagging, hardening, and the like of the skin can be suppressed. Thus, the composition according to the present invention is preferably an anti-aging cosmetic, and more preferably an anti-wrinkle cosmetic.

In one preferred embodiment, the composition according to the present invention is an elastase activity inhibitor.

(A) Cyclic Carboxamide Derivative or Salt Thereof
   The composition according to the present invention comprises a cyclic carboxamide derivative represented by Formula (1) or a salt thereof (hereinafter, sometimes referred to as a component (A), and the same applies to other components). 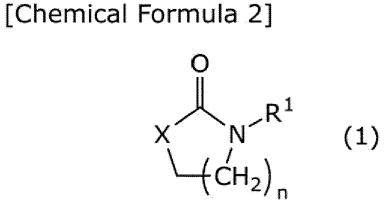 In the formula,
   R¹ is a hydrocarbon group having 1 to 6 carbon atoms which can be substituted with a hydroxy group, or a hydrogen atom,
   X is -CH₂- or -N(R²)-, where R² is a hydrocarbon group having 1 to 6 carbon atoms which can be substituted with a hydroxy group, or a hydrogen atom, and
   n is an integer of 1 to 3.

The hydrocarbon group is not particularly limited, can be, for example, an alkyl group, a cycloalkyl group, an alkenyl group, an alkynyl group, a cycloalkylalkyl group, a haloalkyl group, an alkoxyalkyl group, or an alkoxycarbonylalkyl group, and is preferably an alkyl group.

In a preferred embodiment, in Formula (1) of the component (A),
R¹ is a hydroxyalkyl group having 1 to 3 carbon atoms,
X is -CH₂- or -NH-, and
n is 1.

Specific examples of the cyclic carboxamide derivative represented by Formula (1) include the following.

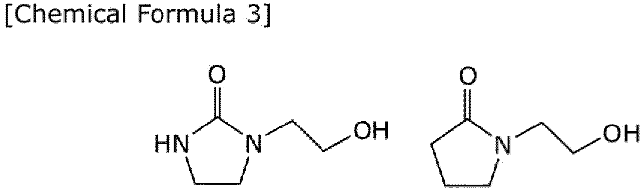

The component (A) is most preferably 1-(2-hydroxyethyl)-2-imidazolidinone.

The component (A) can be a salt of the cyclic carboxamide derivative represented by Formula (1). A kind of salt is not particularly limited as long as it is a pharmacologically acceptable salt, and can be an inorganic salt or an organic salt. Examples of the inorganic salt include a hydrochloride, a sulfate, a phosphate, a hydrobromide, a sodium salt, a potassium salt, a magnesium salt, a calcium salt, a magnesium salt, and an ammonium salt. Examples of the organic salt include an acetate, a lactate, a maleate, a fumarate, a tartrate, a methanesulfonate, a p-toluenesulfonate, a triethanolamine salt, and an amino acid salt.

The component (A) can be used alone or can be used in combination of two or more kinds thereof. The blending amount of the component (A) is preferably 7.5 to 200 mg/mL, more preferably 10 to 180 mg/mL, still more preferably 15 to 150 mg/mL, still further preferably 50 to 150 mg/mL, and particularly preferably 100 to 140 mg/mL with respect to the total amount of the composition.

### (B) Adenosine, Adenosine Derivative or Salt Thereof

The composition according to the present invention comprises (B) adenosine, an adenosine derivative or a salt thereof. Adenosine is one of nucleosides and includes adenine in the base moiety. Examples of the component (B) include adenosine, adenosine 5'-phosphate or a salt of adenosine 5'-phosphate, and adenosine is preferable.

In the case of a salt of an adenosine derivative, a kind of the salt is not particularly limited, as long as it is a pharmacologically acceptable salt, and can be an inorganic salt or an organic salt, and examples thereof include a sodium salt, a potassium salt, and a calcium salt. As a salt of an adenosine derivative, a hydrate thereof can also be used.

The blending amount of the component (B) is preferably 0.5 to 10 mg/mL, more preferably 1 to 10 mg/mL, still more preferably 3 to 10 mg/mL, and still further preferably 5 to 9 mg/mL with respect to the total amount of the composition.

The blending amount of the component (A) with respect to the blending amount of the component (B) ((A)/(B)) is preferably 0.5 to 300 and more preferably 1 to 30 in terms of a mass ratio.

### (C) Water

The cosmetic according to the present invention can comprise (C) water. As the water, water used for cosmetics, quasi-drugs, and the like can be used, and for example, purified water, ultrapure water, ion-exchanged water, tap water, and the like can be used.

In addition to the above components, optional components usually used for cosmetics and pharmaceuticals can be blended into the cosmetic according to the present invention. Examples of the optional components include a humectant, a lower alcohol, a thickener, a surfactant, a sequestering agent, a neutralizing agent, a pH adjusting agent, an antioxidant, a preservative, a drug, an ultraviolet absorber, a powder component, an oily component, and a fragrance, and one kind or two or more kinds thereof can be blended as long as the effect of the present invention is exhibited.

A dosage form of the composition according to the present invention is not particularly limited, and can be any dosage form such as a solution system, a solubilizing system, an emulsifying system, a powder dispersion system, a water-oil bilayer system, a water-oil-powder trilayer system, an ointment, a gel, or an aerosol. In addition, the use form is also not particularly limited, and for example, can be any form such as a lotion, an emulsion, a cream, an essence, a jelly, a gel, an ointment, a pack, a mask, or a foundation.

The composition according to the present invention can be produced according to a conventional method.

### [Examples]

The present invention will be specifically described based on the following examples, but the present invention is not limited to these examples.

### [Preparation of Composition]

1-(2-Hydroxyethyl)-2-imidazolidinone as the component (A) and adenosine as the component (B) were added to ultrapure water so as to satisfy the blending amounts shown in Table 1, and stirred to prepare compositions of Examples 101 to 104 and Comparative Examples 101 to 108.

### [Evaluation of Elastase Inhibitory Activity Effect]

The effect of the compositions of Examples 101 to 104 and Comparative Examples 101 to 108 on elastase activity using N-succinyl-Ala-Ala-Ala-p-nitroanilide as a substrate was evaluated by the following procedure.

50 µL of each composition of Examples and Comparative Examples or a control (ultrapure water was used as a control), 50 µL of 1.25 µg/mL elastase enzyme (CAS No. 39445-21-1, Sigma-Aldrich) solution, and 100 µL of N-succinyl-Ala-Ala-Ala-p-nitroanilide (CAS No. 52299-14-6, Sigma-Aldrich) solution were added to a 96 well plate, and the plate was shaken at 270 rpm for 30 seconds, and then incubated at 37°C for 15 minutes. For the blank, 0.05 M Tris-HCl buffer was used as a substitute for the elastase enzyme. Three wells were used for one treatment group. The 96 well plate was shaken at 270 rpm for 10 seconds to uniformly disperse pigments in the well, and then the absorbance at 415 nm (OD₄₁₅) was measured using a microplate reader.

The elastase inhibitory activity rates of Examples and Comparative Examples were calculated by the following formula. Elastase inhibitory activity rate (%) = {(C - CB) - (S - SB)}/ (C - CB) × 100

In the formula,
C: OD₄₁₅ of control,
CB: OD₄₁₅ of blank of control,
S: OD₄₁₅ of each composition of Examples and Comparative Examples, and
SB: OD₄₁₅ of blank of each composition of Examples and Comparative

### Examples.

The obtained results are shown in Table 1.

### [Significant Difference Test]

For each evaluation, control and each composition of Examples and Comparative Examples were subjected to a significant difference test with unpaired t-test. For all tests, the significance level was less than 5% on both sides. P values are shown in Table 1.

**[Table 1]**

| Table 1 | | (A) mg/mL | (B) mg/mL | Elastase inhibitory activity rate (%) | | | | | | P value |
|---|---|---|---|---|---|---|---|---|---|---|
| | | | | 1 | 2 | 3 | mean | ± | s.d. | |
| Example | 101 | 15 | 1 | 5.0 | 11.6 | 9.8 | 8.8 | ± | 3.4 | P< 0.05 |
| | 102 | 30 | 2 | 15.7 | 23.3 | 18.6 | 19.2 | ± | 3.8 | P< 0.01 |
| | 103 | 60 | 4 | 26.8 | 28.7 | 27.2 | 27.6 | ± | 1.0 | P< 0.001 |
| | 104 | 120 | 8 | 44.9 | 45.3 | 45.8 | 45.3 | ± | 0.5 | P< 0.001 |
| Comparative Example | 101 | 15 | - | 3.0 | 3.8 | 8.4 | 5.1 | ± | 2.9 | - |
| | 102 | 30 | - | 13.4 | 14.6 | 17.2 | 15.1 | ± | 2.0 | P< 0.05 |
| | 103 | 60 | - | 18.7 | 20.6 | 21.7 | 20.3 | ± | 1.5 | P< 0.01 |
| | 104 | 120 | - | 38.8 | 41.0 | 41.3 | 40.4 | ± | 1.4 | P< 0.001 |
| | 105 | - | 1 | -9.7 | 0.0 | -1.1 | -3.6 | ± | 5.3 | - |
| | 106 | - | 2 | -9.7 | -0.1 | -3.0 | -4.3 | ± | 5.0 | - |
| | 107 | - | 4 | -11.4 | -0.8 | -2.9 | -5.1 | ± | 5.6 | - |
| | 108 | - | 8 | -12.1 | -9.2 | -7.7 | -9.7 | ± | 2.2 | - |

### [Formulation Examples 1 to 7]

Formulation examples of compositions according to the invention are shown in the following table. The numerical values in the table are shown in terms of % by mass.

**[Table 2]**

| Component | Formulation Example 1 |
|---|---|
| 1-(2-Hydroxyethyl)-2-imidazolidinone | 1.5 |
| Adenosine | 0.1 |
| Acrylates/C10-30 Alkyl Acrylate Crosspolymer | 0.05 |
| Mineral Oil | 1.5 |
| Dimethicone | 1 |
| Cetyl Ethylhexanoate | 1 |
| Phytosteryl Macadamiate | 0.01 |
| Diphenylsiloxy Phenyl Trimethicone | 0.5 |
| Water | Balance |
| PEG/PPG-17/4 Dimethyl Ether | 0.1 |
| PEG/PPG-14/7 Dimethyl Ether | 0.05 |
| Nicotinic acid amide | 5 |
| Glyceryl Stearate | 0.25 |
| PEG-60 Glyceryl Isostearate | 0.15 |
| Ethanol | 5 |
| Glycerin | 8 |
| BG | 0.03 |
| DPG | 9 |
| Menthoxypropanediol | 0.04 |
| Erythritol | 0.05 |
| Xanthan Gum | 0.05 |
| Carbomer | 0.22 |
| Potassium Hydroxide | 0.08 |
| Dipotassium Glycyrrhizate | 0.05 |
| Rosemary Leaf Oil | 0.02 |
| Lavender Oil | 0.01 |
| Glutamic Acid | 0.01 |
| Eucalyptus Oil | 0.01 |
| Green Tea Extract | 0.01 |
| Potentilla Erecta Root Extract | 0.01 |
| Angelica Keiskei Leaf/Stem Extract | 0.01 |
| Aloe Barbadensis Leaf Extract | 0.01 |
| EDTA-2Na | 0.02 |
| Sodium Metabisulfite | 0.01 |
| Tocopherol | 0.01 |
| Phenoxyethanol | 0.5 |
| Fragrance | 0.06 |

**[Table 3]**

| Component | Formulation Example 2 |
|---|---|
| 1-(2-Hydroxyethyl)-2-imidazolidinone | 1.5 |
| Adenosine | 0.1 |
| Acrylates/C10-30 Alkyl Acrylate Crosspolymer | 0.02 |
| Cetyl Ethylhexanoate | 6 |
| Hydrogenated Polydecene | 6 |
| Dimethicone | 5 |
| Squalane | 3 |
| Stearyl Alcohol | 2 |
| Glyceryl Stearate | 2 |
| Shea Butter | 2 |
| Behenyl Alcohol | 1 |
| Isostearic Acid | 0.5 |
| Hydrogenated Palm Oil | 0.5 |
| Palm Kernel Oil | 0.3 |
| Palm Oil | 0.2 |
| Water | Balance |
| PEG/PPG-17/4 Dimethyl Ether | 0.1 |
| PEG/PPG-14/7 Dimethyl Ether | 0.05 |
| Nicotinic acid amide | 5 |
| PEG-60 Glyceryl Isostearate | 2 |
| Ethanol | 0.04 |
| Glycerin | 3 |
| BG | 6 |
| Xylitol | 5 |
| Xanthan Gum | 0.1 |
| Sodium Polyacrylate | 0.01 |
| Potassium Hydroxide | 0.02 |
| 2-O-Ethyl Ascorbic Acid | 0.05 |
| Prunus Speciosa Leaf Extract | 0.01 |
| Angelica Acutiloba Root Extract | 0.01 |
| Citrus Depressa Peel Extract | 0.01 |
| Iris Florentina Root Extract | 0.01 |
| Eucheuma Serra/Grateloupia Sparsa/Saccharina Angustata/Ulva Linza/Undaria Pinnatifida Extract | 0.01 |
| Typha Angustifolia Spike Extract | 0.01 |
| Isodonis Japonicus Leaf/Stalk Extract | 0.01 |
| Camellia Japonica Seed Extract | 0.01 |
| Saccharina Angustata/Undaria Pinnatifida Extract | 0.01 |
| Green Tea extract | 0.01 |
| Hydrolyzed Silk | 0.01 |
| Bupleurum Falcatum Root Extract | 0.01 |
| Nasturtium Officinale Leaf/Stem Extract | 0.01 |
| Hydrolyzed Conchiolin | 0.01 |
| Cinnamomum Cassia Bark Extract | 0.01 |
| EDTA-2Na | 0.03 |
| Sodium Metabisulfite | 0.02 |
| Sodium Metaphosphate | 0.01 |
| Tocopherol | 0.01 |
| Citric Acid | 0.01 |
| Phenoxyethanol | 0.5 |
| Chlorphenesin | 0.2 |
| Silica | 3 |
| Mica | 0.5 |
| Titanium Oxide | 0.5 |
| Iron Oxide | 0.01 |
| Fragrance | 0.2 |

**[Table 4]**

| Component | Formulation Example 3 |
|---|---|
| 1-(2-Hydroxyethyl)-2-imidazolidinone | 1.5 |
| Adenosine | 0.1 |
| Disteardimonium Hectorite | 0.8 |
| PEG-10 Dimethicone | 2 |
| Hydrogenated Polydecene | 8 |
| Dimethicone | 3 |
| Diphenylsiloxy Phenyl Trimethicone | 3 |
| Triethylhexanoin | 12 |
| Cetyl Ethylhexanoate | 6 |
| Water | Balance |
| Ethanol | 3 |
| Glycerin | 3 |
| DPG | 2 |
| BG | 2 |
| Citric Acid | 0.2 |
| Sodium Citrate | 0.8 |
| Phenoxyethanol | 0.5 |
| Chlorphenesin | 0.2 |

**[Table 5]**

| Component | Formulation Example 4 |
|---|---|
| 1-(2-Hydroxyethyl)-2-imidazolidinone | 1.5 |
| Adenosine | 0.1 |
| PEG-12 Dimethicone | 1 |
| Hydrogenated Polydecene | 2 |
| Dimethicone | 1 |
| Triethylhexanoin | 1 |
| Water | Balance |
| Ethanol | 8 |
| Dipropylene Glycol | 5 |
| Sodium Methyl Stearoyl Taurate | 0.01 |
| Xanthan Gum | 0.2 |
| Carbomer | 0.25 |
| Glycerin | 5 |
| Potassium Hydroxide | 0.18 |
| Phenoxyethanol | 0.35 |
| EDTA-2Na | 0.1 |

**[Table 6]**

| Component | Formulation Example 5 |
|---|---|
| 1-(2-Hydroxyethyl)-2-imidazolidinone | 1.5 |
| Adenosine | 0.1 |
| Behenic Acid | 0.6 |
| Behenyl Alcohol | 2.5 |
| PEG-60 Glyceryl Isostearate | 0.5 |
| PEG-10 Dimethicone | 0.5 |
| Hydrogenated Polyisobutene | 1 |
| Triethylhexanoin | 3 |
| Phytosteryl/Octyldodecyl Lauroyl Glutamate | 2 |
| Diphenylsiloxy Phenyl Trimethicone | 5 |
| Dimethicone | 3 |
| Hydrogenated Palm Oil | 0.5 |
| Palm Kernel Oil | 0.3 |
| Palm Oil | 0.3 |
| Water | Balance |
| Batyl Alcohol | 1 |
| Nicotinic acid amide | 5 |
| Potassium Hydroxide | 0.1 |
| Dimethylacrylamide/Sodium Acryloyldimethyltaurate Crosspolymer | 0.5 |
| PEG-240/HDI Copolymer Bis-Decyltetradeceth-20 Ether | 0.1 |
| Xanthan Gum | 0.05 |
| Glycerin | 15 |
| DPG | 10 |
| BG | 10 |
| PEG/PPG-14/7 Dimethyl Ether | 1 |
| Ethanol | 3 |
| EDTA-2Na | 0.03 |
| Citric Acid | 0.1 |
| Sodium Metaphosphate | 0.1 |
| Sodium Metabisulfite | 0.01 |
| Phenoxyethanol | 0.5 |
| Tocopherol | 0.1 |
| Bupleurum Falcatum Root Extract | 0.1 |
| Cinnamomum Cassia Bark Extract | 0.1 |
| Typha Angustifolia Spike Extract | 0.1 |
| Isodonis Japonicus Leaf/Stalk Extract | 0.1 |
| 2-O-Ethyl Ascorbic Acid | 0.1 |
| Hydrolyzed Silk | 0.1 |
| Camellia Japonica Seed Extract | 0.1 |
| Angelica Acutiloba Root Extract | 0.1 |
| Prunus Speciosa Leaf Extract | 0.1 |
| Green Tea Extract | 0.1 |
| Hydrolyzed Conchiolin | 0.1 |
| Citrus Depressa Peel Extract | 0.1 |
| Iris Florentina Root Extract | 0.1 |
| Eucheuma Serra/Grateloupia Sparsa/Saccharina Angustata/Ulva Linza/Undaria Pinnatifida Extract | 0.05 |
| Saccharina Angustata/Undaria Pinnatifida Extract | 0.05 |
| Nasturtium Officinale Leaf/Stem Extract | 0.1 |
| Aluminum Hydroxide | 0.2 |
| Mica | 1.5 |
| Titanium Oxide | 1.5 |
| Silica | 0.5 |
| Iron Oxide | 0.01 |
| Fragrance | 0.2 |

**[Table 7]**

| Component | Formulation Example 6 |
|---|---|
| 1-(2-Hydroxyethyl)-2-imidazolidinone | 1.5 |
| Adenosine | 0.1 |
| Acrylamides/DMAPA Acrylates/Methoxy PEG Methacrylate Copolymer | 0.25 |
| Pentaerythrityl Tetraethylhexanoate | 2 |
| Isohexadecane | 3 |
| Dimethicone | 3 |
| Water | Balance |
| Ethanol | 4.1 |
| Glycerin | 4 |
| Carbomer | 0.2 |
| Potassium Hydroxide | 0.1 |
| Sodium Metabisulfite | 0.003 |
| EDTA-2Na | 0.02 |
| Phenoxyethanol | 0.5 |

**[Table 8]**

| Component | Formulation Example 7 |
|---|---|
| 1-(2-Hydroxyethyl)-2-imidazolidinone | 1.5 |
| Adenosine | 0.1 |
| Hydrogenated Lecithin | 0.1 |
| Isododecane | 2 |
| Cetyl Ethylhexanoate | 1 |
| Dimethicone | 1 |
| Water | Balance |
| PEG-30 Phytosterol | 0.5 |
| Glycerin | 7 |
| BG | 10 |
| Carbomer | 0.1 |
| Potassium Hydroxide | 0.05 |
| Phenoxyethanol | 0.5 |
| EDTA-2Na | 0.02 |

## Claims

1. An external-use skin preparation composition comprising:
(A) a cyclic carboxamide derivative represented by Formula (1) or a salt thereof 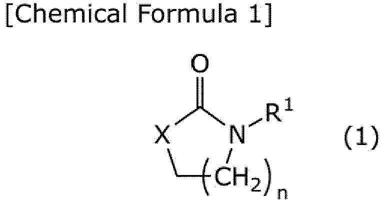 (in the formula,
R¹ is a hydrocarbon group having 1 to 6 carbon atoms which can be substituted with a hydroxy group, or a hydrogen atom,
X is -CH₂- or -N(R²)-, where R² is a hydrocarbon group having 1 to 6 carbon atoms which can be substituted with a hydroxy group, or a hydrogen atom, and
n is an integer of 1 to 3); and
(B) adenosine, an adenosine derivative or a salt thereof.

2. The composition according to claim 1,
wherein, in Formula (1) of the component (A),
R¹ is a hydroxyalkyl group having 1 to 3 carbon atoms,
X is -CH₂- or -NH-, and
n is 1.

3. The composition according to claim 1 or 2, wherein the component (A) is 1-(2-hydroxyethyl)-2-imidazolidinone.

4. The composition according to claim 1 or 2, wherein a blending amount of the component (A) is 7.5 to 200 mg/mL.

5. The composition according to claim 1 or 2, wherein the component (B) is adenosine, adenosine 5'-phosphate, or a salt of adenosine 5'-phosphate.

6. The composition according to claim 1 or 2, wherein the component (B) is adenosine.

7. The composition according to claim 1 or 2, wherein a blending amount of the component (B) is 0.5 to 10 mg/mL.

8. The composition according to claim 1 or 2, which is an anti-aging cosmetic.

9. The composition according to claim 1 or 2, which is an anti-wrinkle cosmetic.

10. The composition according to claim 1 or 2, wherein the composition has elastase inhibitory activity.
